# EUROPEAN PATENT APPLICATION

(11) **EP 4 726 755 A1**
(43) Date of publication of application: **15.04.2026**
(21) Application number: 25206625.3
(22) Date of filing: 03.10.2025
(51) Int. Cl.: H01G 4/228, H01G 4/242, H01G 4/35, A61N 1/375

(54) **GROUND CONNECTION MADE BY A NOTCH IN THE OUTER DIAMETER OF A CERAMIC INSULATOR FOR AN INTERNALLY GROUNDED FILTER FEEDTHROUGH**

(30) Priority: 04.10.2024 US 202463703224 P; 30.09.2025 US 202519344798
(71) Applicant: Greatbatch Ltd., Clarence, NY 14031 (US)
(72) Inventor: CALAMEL, Jonathan, Clarence, NY 14031 (US); HARDENBROOK, Kurt, Kendall, NY 14476 (US); ZABEL, Zachary, Buffalo, NY 14224 (US)
(74) Representative: Mathys & Squire

(57) **Abstract**

An internally grounded filter feedthrough having a filter capacitor connected to a feedthrough is described. The feedthrough comprises an insulator having an active conductive pathway extending to its device and body fluid sides, and a ground conductive pathway extending part-way through the insulator thickness from the insulator device side. An undercut extending into the insulator sidewall communicates with the ground conductive pathway but does not extend to the insulator device or body fluid sides or to the active conductive pathway. A braze seals the insulator sidewall to the ferrule and extends into the undercut so that the braze contacts the ground conductive pathway. That way, the braze provides a ground electrical path from a capacitor ground via to the ground conductive pathway in the insulator connected to the braze sealed to the ferrule.

## Description

### CROSS-REFERENCE TO RELATED APPLICATION

This application claims priority to U.S. provisional application Serial No. 63/703,224, filed on October 4, 2024, as well as U.S. Patent Application Serial No. 19/344,798, filed on 30 September 2025.

### FIELD OF THE INVENTION

The present invention relates to the field of medical devices, particularly active implantable medical devices (AIMD). More particularly, the present invention relates a hermetic internally grounded filter feedthrough for an active implantable medical device. The internally grounded filter feedthrough has a filled via hole providing an electrically conductive pathway through the insulator for the feedthrough.

### BACKGROUND OF THE INVENTION

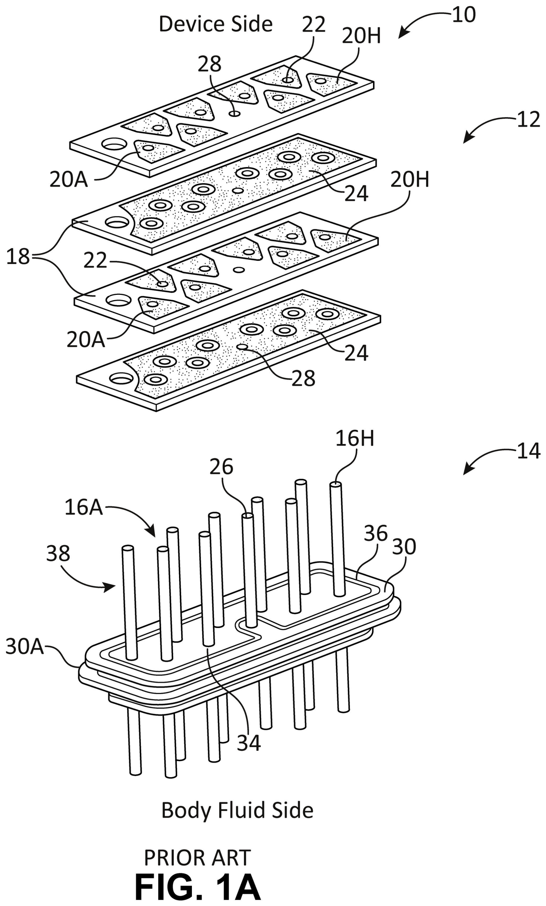

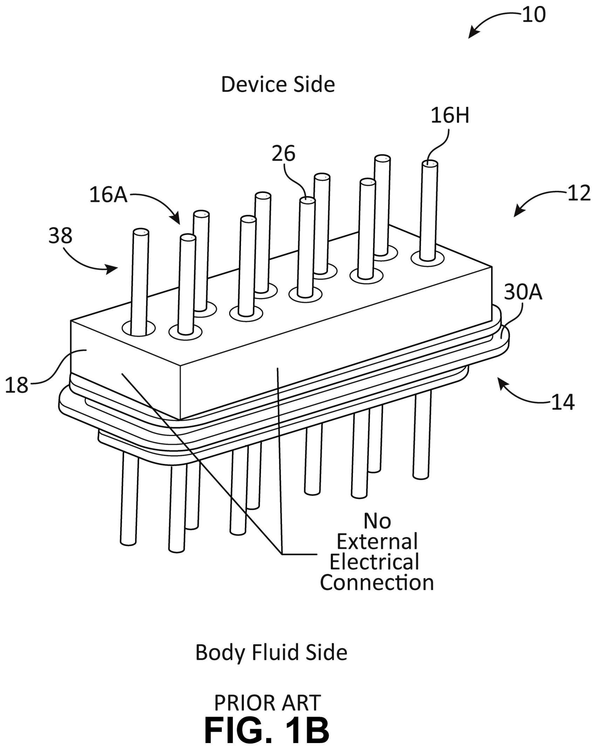

FIGS. 1A and 1B illustrate an internally grounded filter feedthrough 10 according to the prior art. The internally grounded filter feedthrough 10 comprises an EMI filter capacitor 12 that is coupled to a hermetic feedthrough 14. The filter capacitor 12 permits passage of relatively low frequency biologic signals along active terminal pins 16A to 16H while shielding and decoupling/attenuating undesired interference signals of typically high frequency to a ferrule connected to the conductive housing for an AIMD. In general, internally grounded filter feedthroughs are known in the prior art with reference to U.S. Pat. Nos. 5,905,627, 6,529,103, and 6,765,780. These patents are assigned to the assignee of the present invention and fully incorporated herein by reference.

A wide assortment of AIMDs are in commercial use and they frequently include an internally grounded filter feedthrough. Such devices include cardiac pacemakers, cardiac defibrillators, cardioverters, neurostimulators, and other devices for delivering or receiving electrical signals to and from a portion of the body. Sensing and stimulating leads extend from the associated implantable medical device to a distal tip electrode or electrodes in contact with body tissue.

The filter capacitor 12 for the internally grounded filter feedthrough 10 has two sets of electrode plates embedded in spaced relation within an insulative dielectric substrate 18, formed typically as a ceramic monolithic structure. These drawings show eight active electrode plates designated 20A to 20H that correspond to the respective active terminal pins 16A to 16H of the feedthrough 14. As will be appreciated by those skilled in the art of filter feedthroughs, the filter capacitor 12 can have less than or more than the eight active electrode plates 20A to 20H that are shown. The active terminal pins 16A to 16H are preferably made of platinum and extend outwardly to the device and body fluid sides of the feedthrough 14.

The active electrode plates 20A to 20H are electrically connected to a metallization at the inner diameter of a cylindrically-shaped active via hole 22 extending through the dielectric substrate 18. One of the active terminal pins 16A to 16H extending outwardly from the device side of the feedthrough 14 resides in a corresponding one of the metallized active via holes 22 where the terminal pin is connected to a corresponding active electrode plate 20A to 20H by a conductive adhesive or a conductive solder filled into the active via hole 22 in the dielectric substrate 18.

Spaced apart and vertically aligned ground electrode plates 24 are coupled to an internal ground terminal pin 26 that extends outwardly from the device side of the feedthrough 14 to reside in a metallized ground via hole 28 in the dielectric substrate 18 of the filter capacitor 12. However, the ground terminal pin 26, which is preferably made of platinum, does not extend to the body fluid side of the feedthrough 14. The ground electrode plates 24 extend to the ground via hole 28 where they are connected to the ground terminal pin 26 by a conductive adhesive or a conductive solder filled into the ground via hole 28. How the terminal pin 26 is grounded will be described in detail hereinafter.

Importantly, the outer perimeter of the dielectric substrate 18 for the filter capacitor 12 is devoid of a metallization. This means that the ground electrode plates 24 do not extend to the perimeter of the dielectric substrate 18. The overlap between the active electrode plates 20A to 20H and the ground electrode plates 24 creates an effective capacitance area (or ECA).

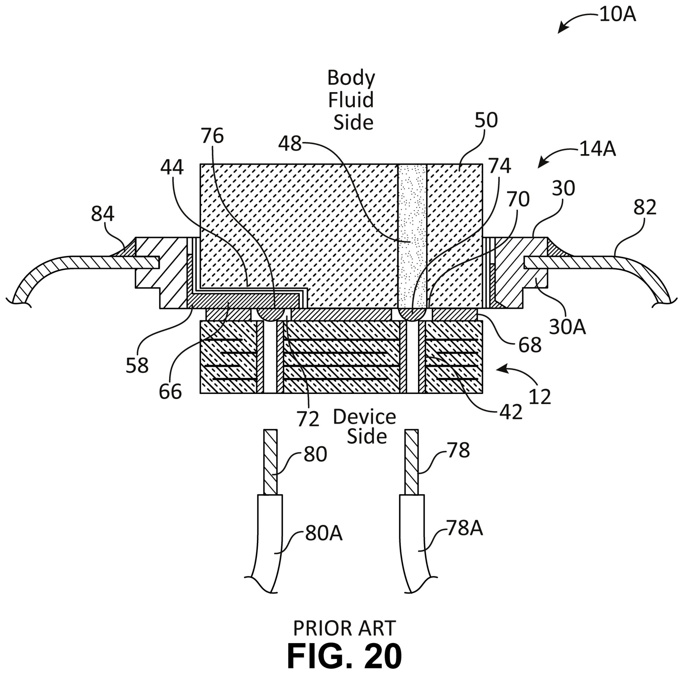

The feedthrough 14 for the internally grounded filter feedthrough 10 is comprised of a ferrule 30 of an electrically conductive metal, preferably titanium. The ferrule 30 comprises an annular sidewall surrounding a ferrule opening. The ferrule sidewall is integrally connected to an outwardly extending flange 30A. As shown in FIG. 20, when the ferrule 30 is sealed in an opening in the housing 82 for an AIMD, the flange 30A is welded 84 to the device housing with the flange edge providing an esthetically contoured transition from the ferrule 30 to the device housing. The ferrule 30 also has a peninsula 32 (FIG. 1A) which extends inwardly into the ferrule opening.

A unitary body of ceramic material serving as an electrically non-conductive insulator 34 is sized and shaped to reside in the ferrule opening. The insulator 34, which includes a recess that matches the outwardly extending ferrule peninsula 32, begins as a unitary green-state substrate or is formed from a plurality of green-state ceramic sheets that are stacked one upon another until a substrate of a desired thickness is obtained. In any event, a ceramic insulator 14 of a desired shape is cut or milled from the green-state substrate before the insulator 14 is subjected to a controlled heating protocol to form a sintered substrate. A suitable material for the insulator 34 is a ceramic, for example, essentially high purity alumina of the chemical formula Al₂O₃ or 3% YSZ. "Essentially pure" means that the post-sintered ceramic is at least 96% alumina up to 99.999% alumina. After sintering, the outer perimeter of the insulator 34 is provided with a metallization (not shown). A gold braze 36 hermetically connects the insulator 34 at this metallization to the ferrule 30 to provide the hermetic feedthrough 14.

The ground terminal pin 26 extends upwardly from the ferrule peninsula 32. With the filter capacitor 12 mounted on the feedthrough 14 (FIG. 1B), the ground terminal pin 26 extends into the ground via hole 28 of the filter capacitor 12 where it is electrically connected to the ground electrode plates 24, which makes it an internally grounded terminal pin 26. Since there is no external metallization on the outer perimeter of the dielectric substrate 18 for the filter capacitor 12, the ground electrode plates 24 are effectively grounded by their connection to the ground terminal pin 26 in turn connected to the ferrule peninsula 32. The feedthrough 14 also includes a platinum telemetry pin 38 that is unfiltered and ungrounded.

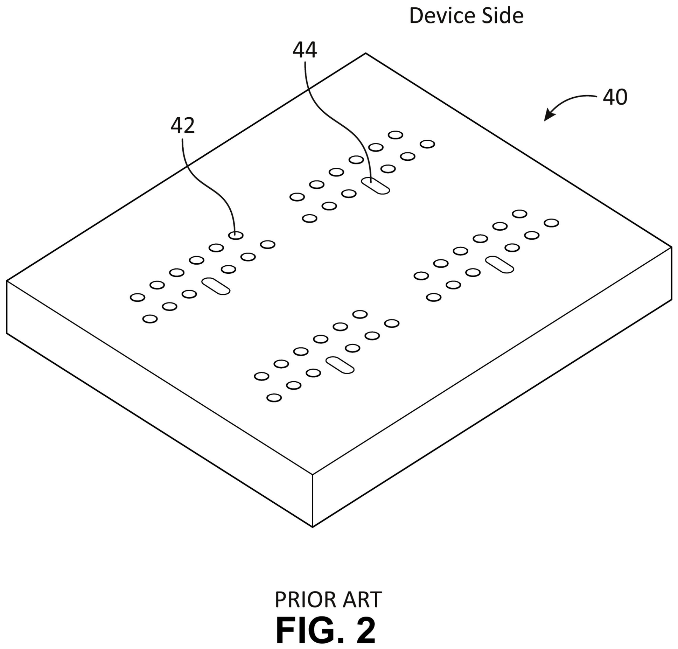

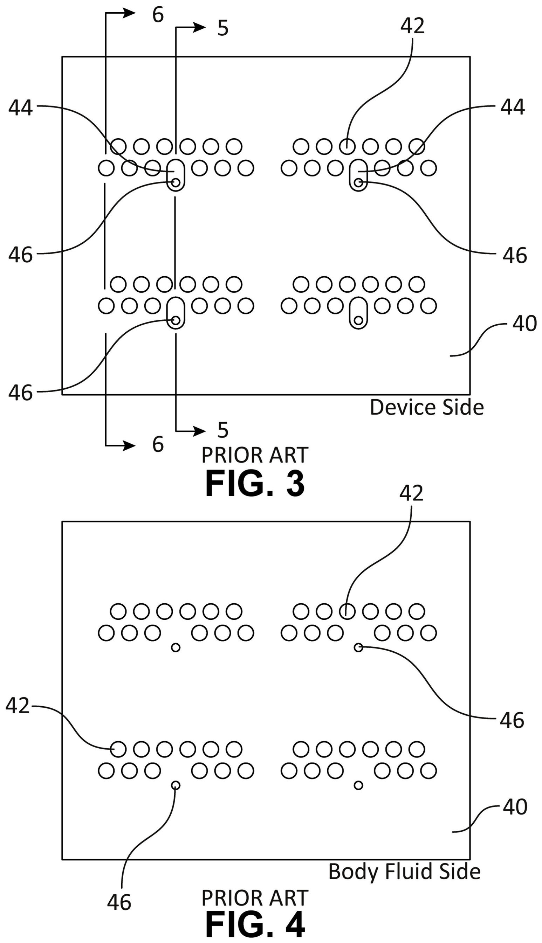

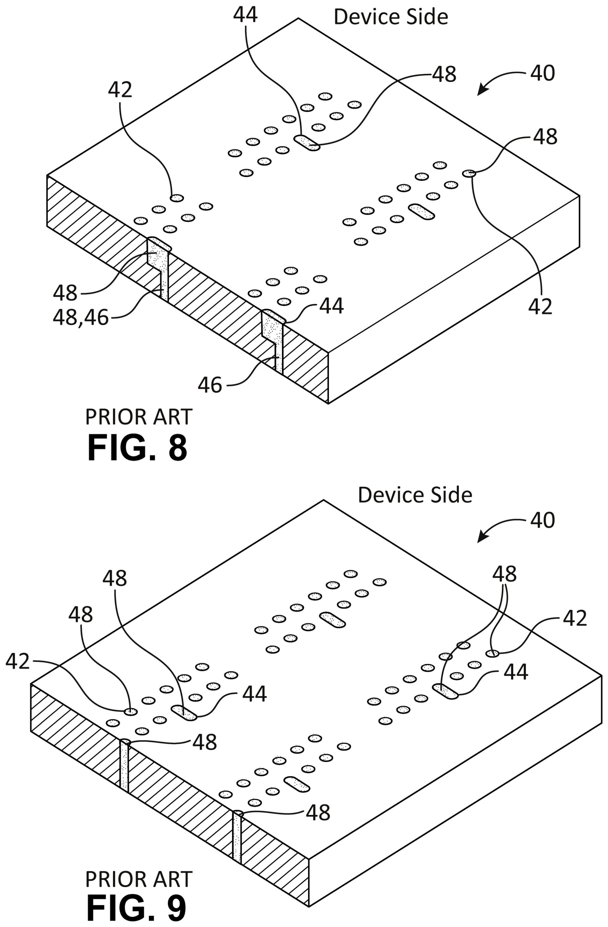

Turning now to FIGS. 2 to 21, these drawings illustrate a substrate 40 of ceramic material which begins as the previously described unitary green-state substrate or stacked green-state ceramic sheets and that is suitable for forming the insulator 34 for the feedthrough 14 illustrated in FIGS. 1A and 1B. Beginning in a green state, this ceramic substrate 40 is comprised principally of alumina and includes several solvents and binders that make it relatively soft and pliable. The ceramic substrate 40 has a thickness which extends from a generally planar device side (FIGS. 2 and 3) to an opposed planar body fluid side (FIG. 4).

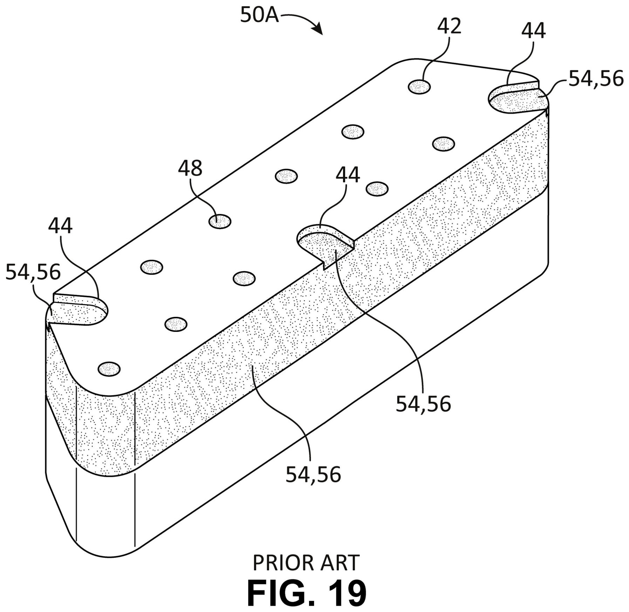

In the green state, the ceramic substrate 40 is easily drilled to provide a patterned cluster of active via holes 42 extending completely through its thickness from the device side to the opposed body fluid side and ground slots 44 that extend only part-way through its thickness from the device side thereof. In the present exemplary embodiment, there are four patterned clusters in the green-state ceramic substrate 40, which will result in four ceramic insulators being cut from the substrate. However, high-density ceramic substrates may produce many more ceramic insulators, for example, over a hundred insulators. Moreover, while each patterned cluster has twelve active via holes 42 and one ground slot 44, that is also exemplary. Instead, any number of active via holes 42 and ground slots 44 may be incorporated into a patterned cluster in the ceramic substrate 40. For example, FIG. 19 shows three ground slots 44. The active via holes 42 in the ceramic substrate 40 may also have counterbores extending from one or both of their device and body fluid sides. In an exemplary embodiment, the ceramic substrate 40 in a green state comprises at least 96% alumina.

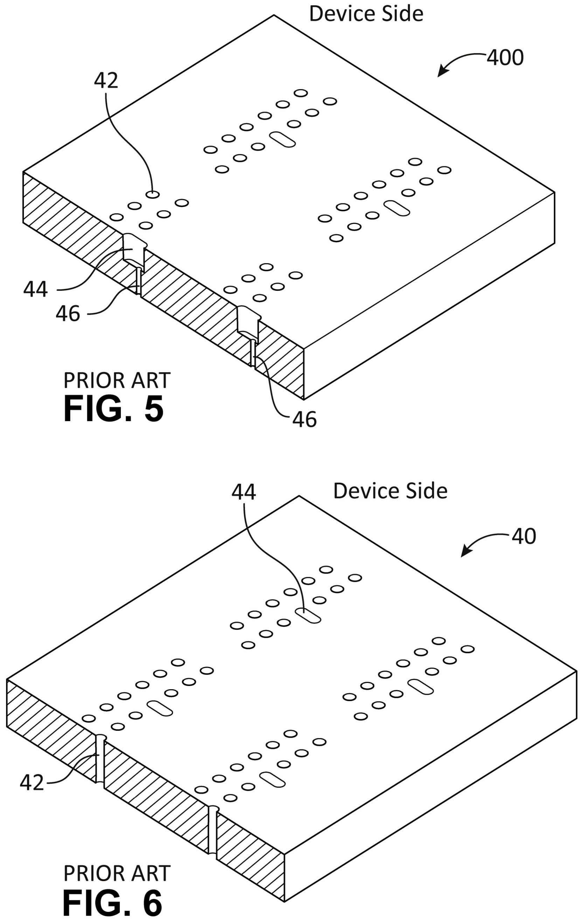

FIGS. 3 to 5 illustrate that each ground slot 44 also includes a relief hole 46 that extends completely through the thickness of the ceramic substrate 40 from its device side to the opposed body fluid side. FIG. 6 shows the device side of the green-state ceramic substrate 40 where the relief hole 46 is not visible. The significance of the relief hole 46 will be described hereinafter.

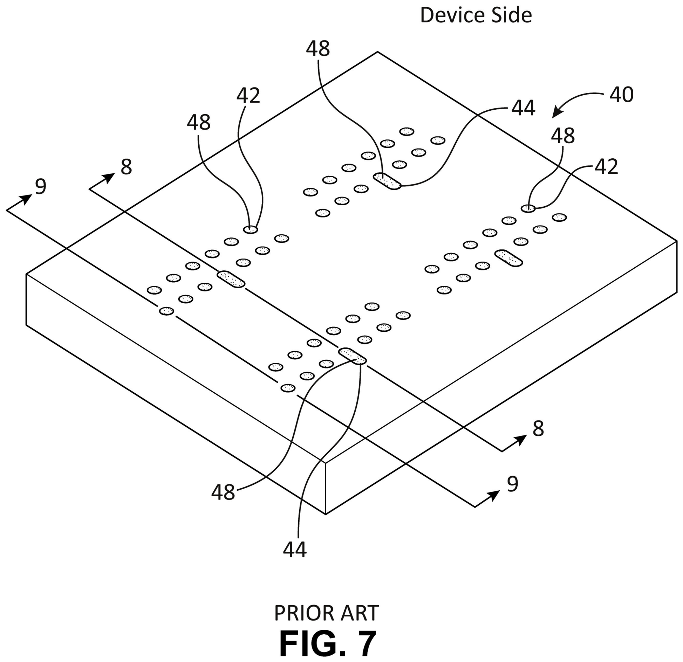

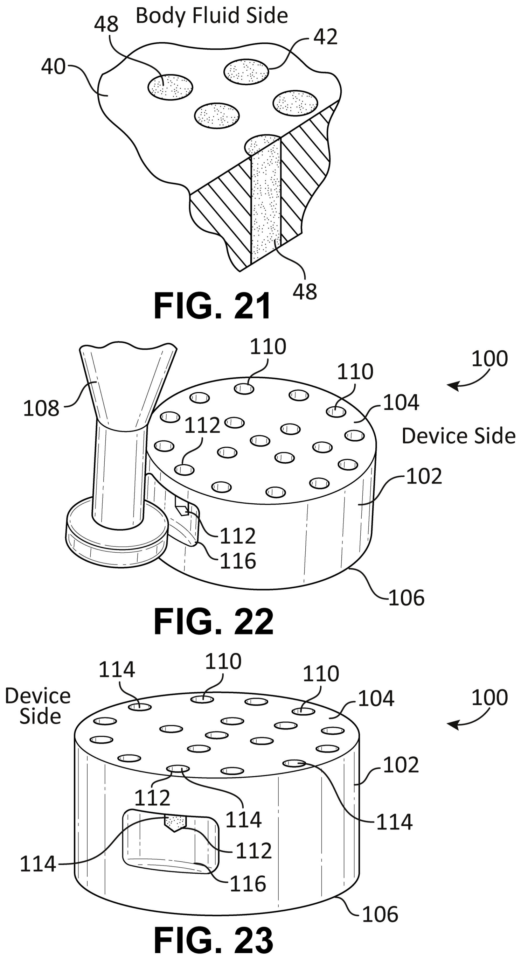

FIG. 7 is the same as FIG. 2 and FIGS. 8 and 9 are the same as respective FIGS. 5 and 6 with the exception that the active via holes 42 and the ground slots 44 including their relief hole 46 have been filled with an electrically conductive platinum-containing material 48 (FIG. 21). For example, the platinum-containing material 48 can be a substantially closed pore, fritless and substantially pure platinum material that fills the active via holes 42 and the ground slots 44 including their relief hole 46.

In lieu of the platinum-containing material 48 being a substantially pure platinum material, the active via holes 42 and the ground slots 44 including their relief hole 46 are filled with a composite reinforced metal ceramic (CRMC) material. The CRMC material is not a substantially pure platinum material, but comprises, by weight %, from about 10:90 ceramic: platinum to about 90:10 ceramic: platinum or, from about 70:30 ceramic: platinum to about 30:70 ceramic: platinum. Examples of suitable ceramic materials for the CRMC include, but are not limited to, alumina (Al₂O₃) or zirconia (ZrO₂) including various stabilized or partially stabilized zirconia like zirconia toughened alumina (ZTA) and alumina toughened zirconia (ATZ) with platinum (Pt) or palladium (Pd).

Preferably, the platinum-containing material 48, whether it is a substantially pure platinum material or the CRMC material, is in the form of a paste having a platinum particle powder or platinum/ceramic particle powder loading ranging from about 20 volume % to about 90 volume % and a viscosity ranging from about 1 x 10⁵ cP to about 1 x 10¹⁰ cP. The platinum-containing material 48 as a paste is a mixture of a substantially pure platinum powder or a platinum/ceramic particle powder, an inactive organic binder, and possibly a solvent and/or plasticizer. Suitable binders are selected from the group consisting of ethyl cellulose, acrylic resin, polyvinyl alcohol, polyvinyl butyral, and a poly(alkylene carbonate) having the general formula R-O-C(=O)-O with R=C₁ to C₅. Poly(ethylene carbonate) or poly(propylene carbonate) are preferred poly(alkylene carbonates). Suitable solvents are selected from the group consisting of terpineol, butyl carbitol, cyclohexanone, n-octyl alcohol, ethylene glycol, glycerol, water, and mixtures thereof.

Suitable methods for filling the active via holes 42 with a paste of platinum-containing material 48 include a vacuum pull, a pressure push, a squeegee fill, among other techniques. In addition, the active via holes 42 and the ground slots 44 including their relief hole 46 must be packed so that the platinum-containing paste occupies at least about 90% of the available space. In a preferred embodiment, the platinum-containing paste occupies about 95% of the available space. In a more preferred embodiment, the platinum-containing paste occupies about 99% of the available space. In the case of the active via holes 42, the platinum-containing material 48 forms platinum-containing conductive pathways 48 extending to the device and body fluid sides of the green-state ceramic substrate 40. The purpose of the relief hole 46 is to prevent the formation of a pressure bubble during filling of the ground slot 44 and relief hole 46 with the platinum-containing material 48.

For additional information regarding via holes filled with electrically conductive materials, reference is made to U.S. Patent Nos. 8,653,384 to Tang et al., 9,492,659 to Tang et al., 10,249,415 to Seitz et al. and RE47,624 to Tang et al. (which is a re-issue of the '384 patent). These patents are assigned to the assignee of the present invention and incorporated herein by reference. For additional information regarding via holes filled with a CRMC material, reference is made to U.S. Patent Nos. 10,350,421 to Seitz et al. and 10,272,252 to Seitz et al. These patents are assigned to the assignee of the present invention and incorporated herein by reference.

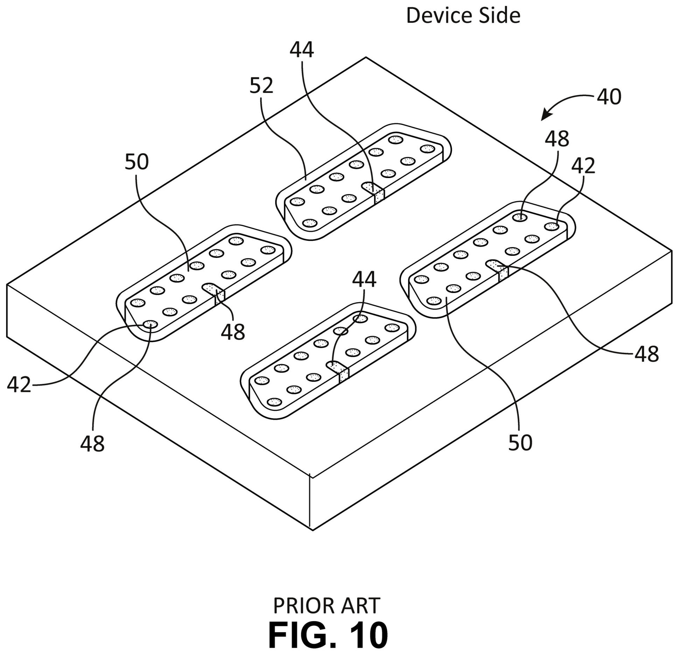

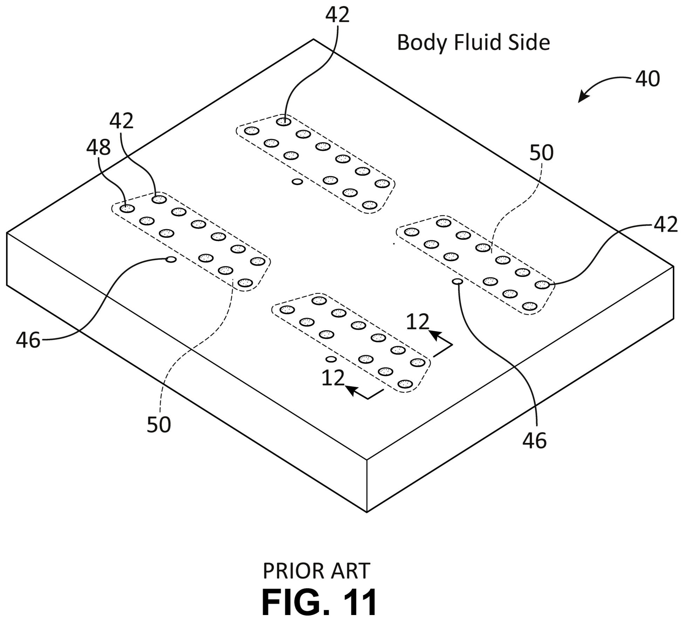

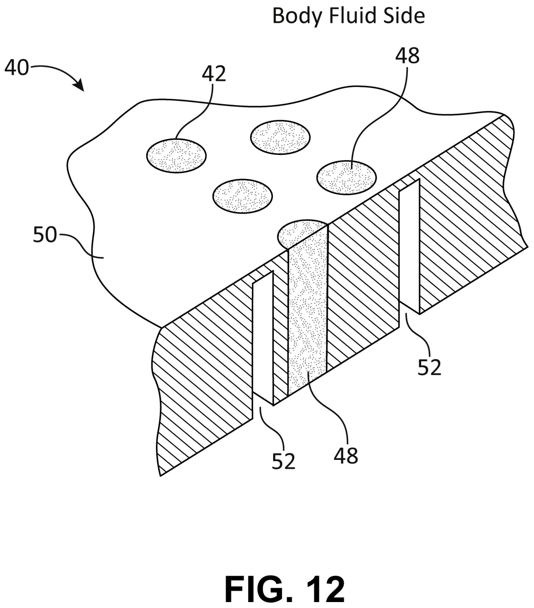

FIGS. 10 and 11 illustrate the green-state ceramic substrate 40 previously described in FIGS. 2 to 9 showing four green-state insulators 50 that have been partially milled 52 out of the substrate 40. This is best understood by referring to the sectional view shown in FIG. 12 where individual green-state insulators 50 have almost been milled 52 out of the ceramic substrate 40. One common technique is to mill a green-state insulator 50 almost all the way out of the ceramic substrate 40 and then simply break it loose from the substrate. This step is known as separating the individual insulator 50 from the ceramic substrate 40. With particular attention to FIG. 11, it is noted that when the individual insulators 50 are cut out of the ceramic substrate 40, the relief hole 46 is eliminated and is not part of the final insulator structure.

After the green-state insulators 50 containing the platinum-containing material 48 in paste form filled into the active via holes 42 and into the ground slots 44 are milled 52 from the ceramic substrate 40, the insulators are exposed to a controlled heating protocol in an ambient air-filled heating chamber. The heating protocol comprises a binder bake-out portion, a sinter portion, and a cool down portion.

In one embodiment, the binder bake-out portion is performed at a temperature of from about 400°C. to about 700°C. for a minimum of about 4 hours. A preferred binder bake-out protocol is performed at a temperature of from about 550°C. to about 650°C. A more preferred binder bake-out is performed at a temperature of from about 500°C. to about 600°C.

Next, the sintering portion of the controlled heating protocol is preferably performed at a temperature ranging from about 1,400°C. to about 1,900°C. for up to about 6 hours. A preferred sintering protocol is at a temperature from about 1,500°C. to about 1,800°C. for up to about 6 hours. A more preferred sintering temperature is from about 1,600°C. to about 1,700°C. for up to about 6 hours.

Then, the cool down portion of the controlled heating protocol occurs either by turning off the heating chamber and allowing the chamber to equalize to room temperature or, preferably by setting the cool down portion at a rate of up to about 5°C./min from the hold temperature cooled down to about 1,000°C. At about 1,000°C., the chamber naturally equalizes to room temperature. A more preferred cool down is at a rate of about 1°C./min from the hold temperature to about 1,000°C. and then allowing the heating chamber to naturally equalize to room temperature. In so doing, a robust hermetic seal is achieved between the sintered ceramic insulator 50 and the sintered platinum-containing conductive pathways 48 in the active via holes 42 and in the ground slots 44.

While the above description regarding the controlled heating protocol has been presented with respect to an alumina ceramic, it is believed that 3% YSZ ceramic will function in a similar manner.

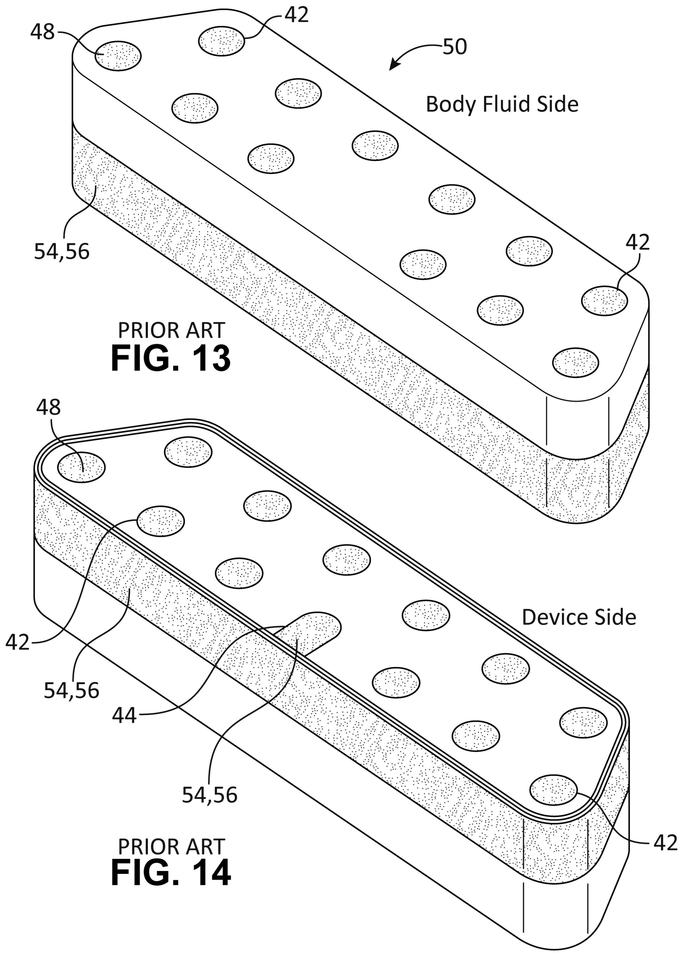

FIGS. 13 and 14 illustrate respective body fluid side and device side views of a sintered insulator 50 after it has been provided with a metallization system for subsequent gold brazing into an opening in a ferrule 30. A suitable metallization comprises two metallization layers, a first adhesion layer 54 that is directly applied to the perimeter of the insulator 50, and a second, wetting layer 56, which is applied on top of the adhesion layer 54. In a preferred embodiment, the adhesion layer 54 is titanium, and the wetting layer 56 is either molybdenum or niobium. However, for the sake of simplicity the adhesion and wetting layers 54, 56 are not shown. FIG. 14 also shows that the sputtered metallization layers 54, 56 extend into the ground slot 44.

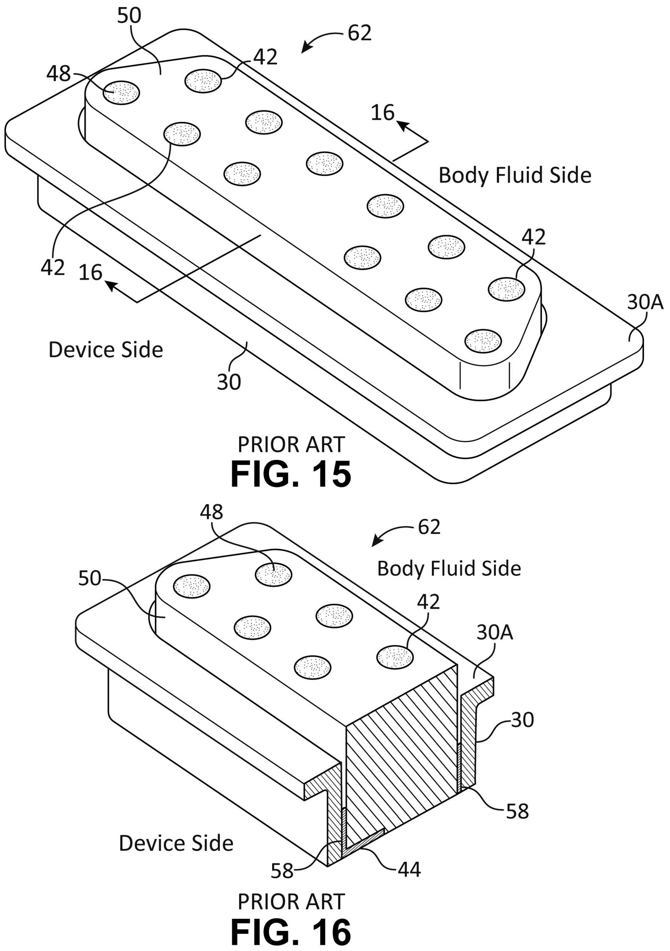

FIG. 15 shows the metallized insulator from FIGS. 13 and 14 after it has been positioned in a suitably shaped opening in the ferrule 30. A ring-shaped gold preform 58 is positioned in the gap residing between the inner surface of the ferrule 30 and the metallization layers 54, 56 contacted to the outer surface of the insulator 50. This assembly is then subjected to a brazing process, as is well known to those skilled in the art related to brazing a ceramic material to a metallic flange. The brazing process melts the gold 58 which causes it to flow into intimate contact with the metallization 54, 56 and the inner surface of the titanium ferrule 30 to thereby form a hermetic seal joining the insulator 50 to the ferrule 30. This results in a feedthrough 62 that is similar to the previously described feedthrough 14 shown in FIGS. 1A and 1B. The difference is that the feedthrough 62 shown in FIGS. 15 to 18 has the sintered platinum-containing conductive pathways 48 (FIG. 21) in the active via holes 42 while the feedthrough 14 shown in FIGS. 1A and 1B includes the platinum terminal pins 16A to 16H, the ground terminal pin 26 and the telemetry pin 38.

FIG. 16 is a cross-sectional view taken from FIG. 15 showing that the gold braze 58 forms a strong mechanical and hermetic seal between the ferrule 30 and the insulator 50 for the feedthrough 62. The gold braze also extends into the ground slot 44. The metallization layers 54, 56 are not shown for simplicity, but are present. In that respect, it is understood that throughout the rest of the drawing figures, if the metallization layers are not shown, they are nonetheless present.

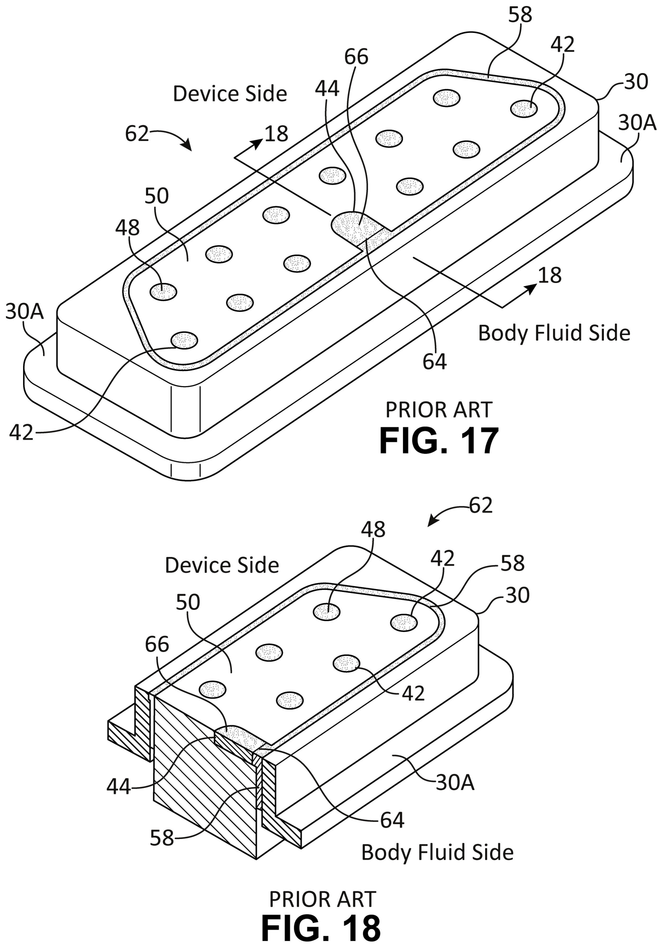

FIGS. 17 and 18 illustrate the device side of the feedthrough 62 of FIG. 16 including the gold braze 58. In general, the surface of the alumina insulator 50 is flush with the top of the ferrule 30, however, it will be appreciated that the insulator 50 can be recessed below the upper surface of the ferrule 30 or stand proud of it. It is preferred that the insulator 50 is flushed to accommodate for mounting a filter capacitor 12 similar to that shown in FIGS. 1A and 1B onto the feedthrough 62.

FIG. 19 illustrates a sintered insulator 50A that is very similar to the insulator 50 shown in FIG. 14 except there are three metallized 54, 56 ground slots 44. It is appreciated that three grounding locations with a gold braze is beneficial as the active lead count increases in response to the demands of modern implantable medical devices becoming smaller with increased functionality. This is known as a multipoint grounding system, and it ensures that each active conductive pathway of a filter feedthrough has a high insertion loss. Insertion loss is a measure of filter performance.

FIG. 20 is a sectional view of an internally grounded filter feedthrough 10A showing the ground slot 44 of FIGS. 13 to 19 filled with a secondary braze 66. The significance of the secondary braze 66 will be described in detail hereinafter.

The internally grounded filter feedthrough 10A comprises an EMI filter capacitor 12 that is coupled to a hermetic feedthrough 14A. The filter capacitor 12 is similar to the capacitor 12 shown in FIGS. 1A and 1B. The feedthrough 14A does not support terminal pins but, instead, its conductive pathways are provided by the sintered platinum-containing conductive material 48 (FIG. 21).

An insulative washer 68 is positioned between the filter capacitor 12 and the feedthrough 14A. The washer 68 has a plurality of openings 70 that are aligned with the sintered platinum-containing conductive pathways 48. A second opening 72 is aligned with the braze 66 residing in the metallized ground slot 44. Electrically conductive solder pads 74 and 76, preferably containing platinum, reside in the respective openings 70, 72. Solder pad 74 provides a conductive path from an active lead wire 78 to the sintered platinum-containing conductive pathway 48 while solder pad 76 provides a conductive path from a ground lead wire 80 to the braze 66 in the metallized ground slot 44. The lead wires 78, 80 are provided with insulation 78A, 80A.

Referring back to FIGS. 17 and 18, these drawings show a demarcation line 64 separating the gold braze 58 surrounding the metallization layers 54, 56 contacted to the outer surface of the insulator 50 and sealing to the inner surface of the ferrule 30 from a secondary braze 66 in the ground slot 44. This demarcation line 64 isn't necessarily a straight line, as shown, but does indicate that due to capillary action during the gold brazing operation, molten primary gold braze 58 tends to flow out of the ground slot 44 and seep to the perimeter of the insulator 50. In some cases, the primary gold braze 58 in the ground slot 44 can become very thin or pull away from the insulator completely. This necessitates that in a secondary low temperature braze operation, an additional braze material 66 is added to adequately fill the ground slot 44. The secondary low-temperature braze 66 does not need to be biocompatible since it is not exposed to body fluids. Accordingly, the secondary braze 66 can consist of TiCuSiI, CuSiI or even a nano-gold material, which would reflow at a lower temperature.

While not shown in the FIG. 20, the lead wires 78, 80 are connected to electronic components that are mounted on a printed circuit board (PCB) assembly contained inside the device housing 82 for an AIMD. That way, electrical continuity is established from the electronic components of the PCB assembly to the body fluid side of the sintered platinum-containing conductive pathway 48 and to the primary and secondary brazes 58, 66 residing in the ground slot 44 where they are grounded to the ferrule 30 connected to the device housing 82. The internally grounded capacitor 12 shown in FIG. 20 is then able to dissipate electrical energy from its lead wires 78, 80 to the ferrule 30.

In that respect, there is a need to design a feedthrough that does not experience gold braze material seeping out of the ground slot 44 and wicking to the perimeter of the insulator 50. Not only is the rework required to add the secondary braze material 66 time consuming and the source of added expense, but the secondary braze 66 can alloy with the platinum comprising the solder pads 74, 76. This is undesirable as it adversely affects the conductivity of the electrical connection between the ground lead wire 80 and the primary and secondary brazes 58, 66 grounded to the ferrule 30.

Therefore, there is a desire to provide an improved internally grounded filter feedthrough where the internal ground connection to the ferrule does not require a secondary braze rework step. It would also be beneficial to provide a ground connection from a lead wire extending from a PCB assembly that is not prone to alloying with the braze materials hermetically sealing the feedthrough insulator to the ferrule.

### SUMMARY OF THE INVENTION

Aspects and embodiments of the present invention are set out in the appended claims.

The present invention generally relates to an insulator having a thickness defined by an insulator sidewall extending to an insulator device side opposite an insulator body fluid side. At least one active conductive pathway extends through the insulator thickness to the device and body fluid sides, and at least one ground conductive pathway extends part-way through the insulator thickness from the insulator device side. An undercut extends into the insulator sidewall so that the undercut is in communication with the ground conductive pathway. The undercut preferably does not extend to the device or body fluid sides of the insulator and preferably nor is it in communication with the active conductive pathway. In one embodiment, the insulator has a plurality of ground conductive pathways, and a plurality of discrete undercuts extending into the insulator sidewall are in communication with a respective one of the plurality of ground conductive pathways. In another embodiment, the insulator has a plurality of ground conductive pathways, and an annular undercut extends into the insulator sidewall in communication with each of the plurality of ground conductive pathways. Further, a metallization may be contacted to the insulator sidewall. The metallization may extend to the device side of the insulator but not to the body fluid side. Importantly, the metallization may extend into the undercut where it contacts the at least one ground conductive pathway.

The present invention further relates to a feedthrough comprising a ferrule defining a ferrule opening extending to a ferrule device side opposite a ferrule body fluid side. An insulator hermetically sealed to the ferrule in the ferrule opening has a thickness defined by an insulator sidewall extending to an insulator device side residing at or adjacent to the ferrule device side and an opposed insulator body fluid side residing at or adjacent to the ferrule body fluid side. At least one active conductive pathway extends through the insulator thickness to the device and body fluid sides, and at least one ground conductive pathway extends part-way through the insulator thickness from the insulator device side. An undercut extends into the insulator sidewall so that the undercut is in communication with the at least one ground conductive pathway, but the undercut is preferably not in communication with the at least one active conductive pathway. Then, a braze hermetically seals the insulator sidewall to the ferrule. The braze may extend into the undercut so that it is in in contact with the at least one ground conductive pathway, but the braze preferably does not extend to the device or body fluid sides of the insulator.

In one embodiment, the insulator has a plurality of ground conductive pathways, and a plurality of discrete undercuts extending into the insulator sidewall are in communication with a respective one of the plurality of ground conductive pathways. In another embodiment, the insulator has a plurality of ground conductive pathways, and an annular undercut extending into the insulator sidewall are in communication with each of the plurality of ground conductive pathways. Further, a metallization may be contacted to the insulator sidewall including the undercut and may be in contact with the at least one ground conductive pathway. Moreover, the metallization extends to the device side of the insulator but does not extend to the body fluid side.

Still further, the present invention relates to an internally grounded filter feedthrough having a feedthrough comprising a ferrule defining a ferrule opening extending to a ferrule device side opposite a ferrule body fluid side. An insulator hermetically sealed to the ferrule in the ferrule opening has a thickness defined by an insulator sidewall extending to an insulator device side residing at or adjacent to the ferrule device side and an opposed insulator body fluid side residing at or adjacent to the ferrule body fluid side. At least one active conductive pathway extends through the insulator thickness to the insulator device and body fluid sides, and at least one ground conductive pathway extends part-way through the insulator thickness from the insulator device side. An undercut extends into the insulator sidewall so that the undercut is in communication with the ground conductive pathway. Then, a braze hermetically sealing the insulator sidewall to the ferrule extends into the undercut so that the braze is in contact with the ground conductive pathway.

The internally grounded filter feedthrough also has a filter capacitor comprising a dielectric substrate supporting at least one active electrode plate interleaved in a capacitive relationship with at least one ground electrode plate. The dielectric substrate has a substate sidewall extending to a first end surface opposite a second end surface. A capacitor active via extends through the dielectric substrate to the first and second end surfaces, and the active electrode plate is electrically connected to the capacitor active via. There is also a capacitor ground via extending through the dielectric substrate to the first and second end surfaces, and the ground electrode plate is electrically connected to the capacitor ground via.

To provide the internally grounded filter feedthrough, the dielectric substrate first end surface is positioned adjacent to the insulator device side with the capacitor active via connected to the active conductive pathway extending through the insulator so that an active electrical path extends from the capacitor active via at the second side of the dielectric substrate to the active conductive pathway at the body fluid side of the insulator. Further, the braze hermetically sealing the insulator sidewall to the ferrule also extends into the insulator undercut to contact the ground conductive pathway so that a ground electrical path extends from the capacitor ground via at the second side of the dielectric substrate to the ground conductive pathway in the insulator connected to the braze sealed to the ferrule.

Importantly, the undercut preferably does not extend to the device or body fluid sides of the insulator and preferably is not in communication with the active conductive pathway in the insulator. A metallization may extend to the device side of the insulator but not to the body fluid side and may be contacted to the insulator sidewall including the undercut and in contact with the ground conductive pathway. An active solder pad connects the active via at the first side of the dielectric substrate to the active conductive pathway at the device side of the insulator, and a ground solder pad connects the ground via at the first side of the dielectric substrate to the ground conductive pathway at the device side of the insulator. There is also an insulative washer that resides between the device side of the insulator and the first side of the dielectric substrate for the filter capacitor.

These and other aspects of the present invention will become increasingly more apparent to those skilled in the art by reference to the following detailed description and to the appended drawings.

### BRIEF DESCRIPTION OF THE DRAWINGS

FIG. 1A illustrates an exploded perspective view of an internally grounded filter feedthrough 10 according to the prior art.
FIG. 1B illustrates the structure of FIG. 1A fully assembled into a filter feedthrough 10 according to the prior art.
FIG. 2 is a perspective view looking at the device side of a green-state ceramic substrate 40 showing four clusters of active via holes 42 and a ground slot 44 according to the prior art.
FIG. 3 is a plan view looking at the device side of the green-state ceramic substrate 40 shown in FIG. 2 according to the prior art.
FIG. 4 is a plan view looking at the body fluid side of the green-state ceramic substrate 40 shown in FIG. 2 according to the prior art.
FIG. 5 is a cross-sectional view taken along line 5-5 of FIG. 3 according to the prior art.
FIG. 6 is a cross-sectional view taken along line 6-6 of FIG. 3 according to the prior art.
FIG. 7 is a perspective view looking at the device side of the green-state ceramic substrate 40 shown in FIG. 2 but with all of the active via holes 42 and the ground slot 44 of each of the four clusters filled with a platinum-containing conductive paste 48 according to the prior art.
FIG. 8 is a cross-sectional view taken along line 8-8 of FIG. 7 according to the prior art.
FIG. 9 is a cross-sectional view taken along line 9-9 of FIG. 7 according to the prior art.
FIG. 10 is a perspective device side view illustrating four individual insulators 50 corresponding to the four clusters having been partially milled out of the green-state ceramic substrate 40 shown in FIGS. 2 to 9 according to the prior art.
FIG. 11 is a perspective body fluid side view illustrating the green-state ceramic substrate 40 shown in FIG. 10 and that the individual insulators 50 have not yet been completely milled out of the substrate 40 according to the prior art.
FIG. 12 is a cross-sectional view taken along line 12-12 of FIG. 11 illustrating the platinum-containing conductive paste 48 filled in an active via hole 42 according to the prior art.
FIG. 13 is a perspective view showing the body fluid side of an insulator 50 after it has been milled and broken out of the green-state ceramic insulator 40 shown in FIGS. 10 and 11 and then provided with a metallization system 54, 56 according to the prior art.
FIG. 14 is a perspective view showing the device side of the insulator 50 shown in FIG. 13 according to the prior art.
FIG. 15 is a perspective view illustrating the body fluid side of the insulator 50 shown in FIGS. 13 and 14 brazed into an opening in the ferrule 30 to provide a feedthrough 62 according to the prior art.
FIG. 16 is a cross-sectional view taken along line 16-16 of FIG. 15 according to the prior art.
FIG. 17 is a perspective view illustrating the device side of the feedthrough 62 shown in FIG. 15 including the gold braze 58 connecting the ceramic insulator 50 to the ferrule 30 according to the prior art.
FIG. 18 is a cross-sectional view taken along line 18-18 of FIG. 17 illustrating a secondary braze 66 in the ground slot 44 in the insulator 50 and the gold braze 58 connecting the insulator to the ferrule 30 according to the prior art.
FIG. 19 is a perspective view of an insulator 50A that is similar to the insulator 50 shown in FIG. 14 but with multiple ground slots 44 according to the prior art.
FIG. 20 is a cross-sectional view showing the secondary braze 66 in the ground slot 44 and the primary gold braze 58 sealing the insulator 50 to the ferrule 30 in an internally grounded filter feedthrough 10A according to the prior art.
FIG. 21 is a sectional perspective view of the body fluid side of an insulator showing a plurality of active via holes 42 filled with a platinum-containing conductive paste 48 according to the prior art.
FIG. 22 is a perspective view of an insulator 100 according to the present invention with a milling tool 108 forming an undercut 116 in its sidewall 102.
FIG. 23 is a perspective view showing the insulator 100 from FIG. 22 with the undercut 116 in communication with a paste of platinum-containing material 114 in a ground hole 112 in the insulator.
FIG. 24 is a side elevational view showing the insulator 100 from FIG. 23 with a metallization 118, 120 contacted to its sidewall 102.
FIG. 25 is a perspective view of the insulator 100 according to the present invention showing two undercuts 116 in communication with ground conductive pathways 114 in respective ground holes 112.
FIG. 26 is an exploded view of an internally grounded filter feedthrough 10 according to the present invention.
FIG. 26A is an enlarged view of the indicated area in FIG. 26.
FIG. 27 is a perspective view of another exemplary insulator 100A according to the present invention showing an annular undercut 116A in the sidewall 102 of the insulator.
FIG. 28 is a side elevational view of the insulator 100A shown in FIG. 26 with the annular undercut 116 in communication with three ground conductive pathways 114.

### DETAILED DESCRIPTION OF THE PREFERRED EMBODIMENTS

As used in this description, the term "via hole" means an open passageway that extends through the thickness of an insulator from a device side to an opposed body fluid side or from a body fluid side to a device side. In contrast, the word "hole" as used in the term "ground hole" is an opening that does not extend completely through the thickness of an insulator. Instead, a ground hole extends part-way through the thickness of an insulator from its device side, but the ground hole does not extend to the body fluid side of the insulator.

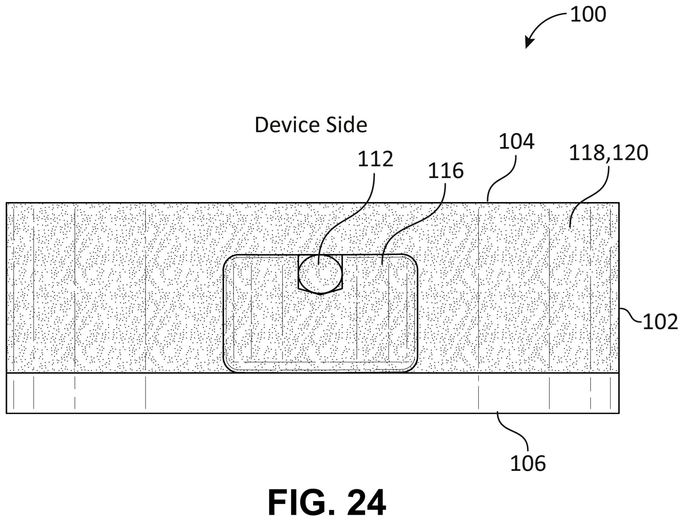

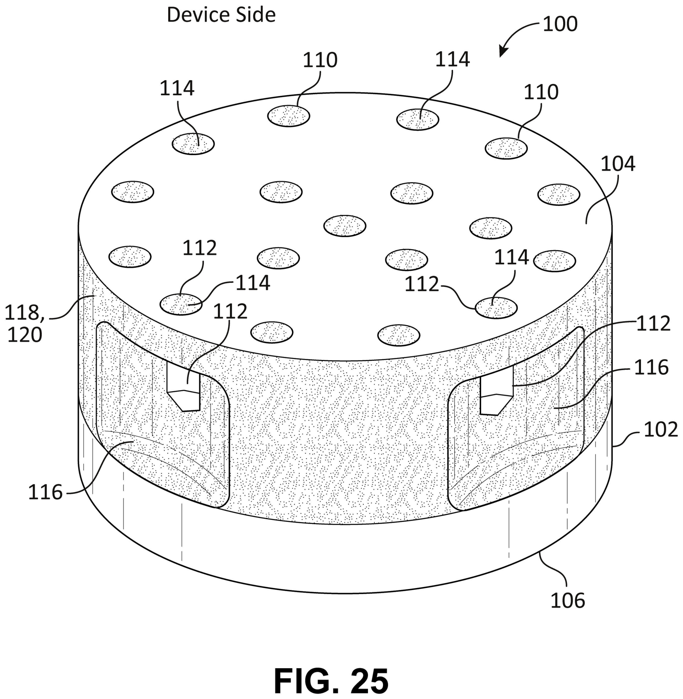

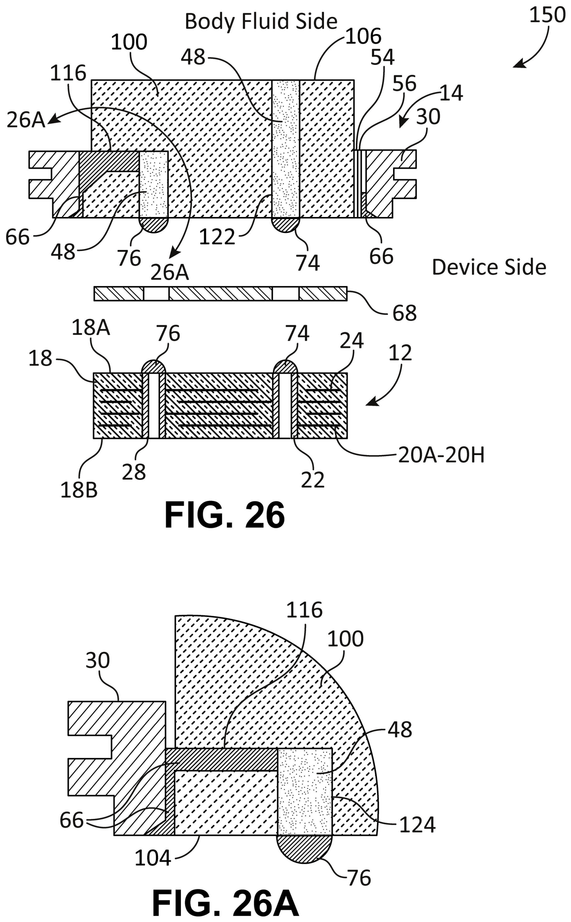

Turning now to FIGS. 22 to 26 and 26A, an exemplary insulator 100 for an internally grounded filter feedthrough 150 (FIG. 26) according to the present invention is illustrated. The insulator 100 has a thickness defined by a perimeter sidewall 102 that extends from a first or device side 104 to a second or body fluid side 106. As is the case with the previously described insulator 50, a milling tool 108 is used to cut or separate the insulator 100 from a substrate of a green-state ceramic material that has a patterned cluster of drilled active via holes 110 extending completely through its thickness from the device side 104 to the opposed body fluid side 106. However, instead of the patterned cluster comprising a ground slot as with the prior art insulator 50, at least one ground hole 112 is drilled part-way through the thickness of the green-state ceramic substrate from the device side 104. This means that the ground hole 112 does not extend to the body fluid side 106 of the ceramic substrate.

The active via holes 110 and the ground hole 112 comprising the patterned cluster in the green-state ceramic substrate are filled with a paste of a platinum-containing material 114 and the exemplary individual insulator 100 is then partially milled from the green-state ceramic substrate using a milling tool 108 (FIG. 22) in a similar manner as previously illustrated with the insulator 50 shown in FIG. 12. During this milling operation, an important aspect of the present invention takes place.

In addition to milling around the perimeter of the clustered pattern of active via holes 110 and the ground hole 112, the milling tool 108 is also used to form a discrete undercut 116 into the sidewall 102 to expose the platinum-containing material 114 in the ground hole 112. The undercut 116 is spaced below the device side 104 of the insulator 100 and above the body fluid side 106 thereof. In FIGS. 22 to 24, since the ground hole 112 does not extend to the body fluid side 106 of the insulator 100, the discrete undercut 116 only exposes an inner portion of the platinum-containing material 114 in the ground hole 112. Preferably, the undercut 116 has a height that extends from about 30% to about 60% of the thickness of the insulator 100. After the green-state ceramic insulator 100 including its discrete undercut 116 is milled almost completely out of the ceramic substrate, it is broken loose from the substrate. This step is known as separating the insulator 100 from the ceramic substrate.

The green-state ceramic insulator 100 is then subjected to a controlled heating protocol to form a sintered insulator. In a similar manner as previously described with respect to the prior art insulators 50, the controlled heating protocol occurs in an ambient air-filled heating chamber and comprises a binder bake-out portion, a sinter portion, and a cool down portion. Not only does the heating protocol transform the green-state ceramic material into a sintered ceramic insulator, but it also transforms the platinum-containing material 114 in the active via holes 110 and in the ground hole 112 into respective active and ground platinum-containing conductive pathways 114.

In a similar manner as described with respect to FIGS. 13 and 14, the sidewall 102 is then provided with a metallized 118, 120. The metallization 118, 120 is also contacted to the discrete undercut 116 and to the exposed surface of the ground conductive pathway in the ground hole 112. The metallized insulator 100 is now ready to be hermetically sealed into an opening in a ferrule 30 with a gold braze 66 as previously described with respect to FIGS. 15 and 16.

As shown in FIGS. 26 and 26A, the result of the brazing process is a feedthrough that is similar to the prior art feedthrough 10A shown in FIG. 20. However, while the prior art feedthrough 10A is susceptible to braze material seeping out of the ground slot 44 and wicking to the perimeter of the insulator 50 where the braze material could possibly alloy with the platinum comprising the solder pads 74, 76, the discrete undercut 116 effectively isolates the gold braze 66 contacting the platinum-containing conductive pathway 114 in the ground hole 112. This isolation prevents gold 66 from reaching the solder pads 74, 76 with the result that the possibility of subsequent alloying and its detrimental effects on electrical conductivity is eliminated.

Thus, the present invention relates to an internally grounded filter feedthrough 150 having a feedthrough 14 comprising a ferrule 30 defining a ferrule opening extending to a ferrule device side opposite a ferrule body fluid side. An insulator 100 hermetically sealed to the ferrule 30 in the ferrule opening has a thickness defined by an insulator sidewall extending to an insulator device side residing at or adjacent to the ferrule device side and an opposed insulator body fluid side residing at or adjacent to the ferrule body fluid side. At least one active conductive pathway 122 extends through the insulator 100 thickness to the insulator device and body fluid sides, and at least one ground conductive pathway 124 extends part-way through the insulator thickness from the insulator device side. A discrete undercut 116 extends into the insulator sidewall so that the undercut is in communication with the ground conductive pathway 124. Then, a braze 66 hermetically sealing the insulator 100 to the ferrule 30 extends into the undercut 116 so that the braze is in contact with the ground conductive pathway 124.

The internally grounded filter feedthrough 150 also has a filter capacitor 12 comprising a dielectric substrate 18 supporting active electrode plates 20A to 20H interleaved in a capacitive relationship with ground electrode plates 24. The dielectric substrate 18 has a substate sidewall extending to a first end surface 18A opposite a second end surface 18B. A metallized active via 22 extends through the dielectric substrate 18 to the first and second end surfaces 17A, 18B, and the active electrode plates 20A to 20H are electrically connected to the capacitor active via 22. There is also a metallized ground via 28 extending through the dielectric substrate 18 to the first and second end surfaces 18A, 18B, and the ground electrode plates 24 are electrically connected to the capacitor ground via 28.

To provide the internally grounded filter feedthrough 150, the dielectric substrate 18 first end surface 18A is positioned adjacent to the insulator device side with the capacitor active via 22 connected to the active conductive pathway 122 extending through the insulator 100 so that an active electrical path extends from the capacitor active via 22 at the second side 18B of the dielectric substrate 18 to the active conductive pathway 122 at the body fluid side of the insulator 100. Further, the braze 66 hermetically sealing the insulator 100 to the ferrule 30 also extends into the insulator undercut 116 to contact the ground conductive pathway 124 so that a ground electrical path extends from the capacitor ground via 28 at the second side 18B of the dielectric substrate 18 to the ground conductive pathway 124 in the insulator 100 connected to the braze 66 sealed to the ferrule 30. Importantly, the undercut 116 does not extend to the device or body fluid sides of the insulator 100 nor is it in communication with the active conductive pathway 122 in the insulator 100.

An active solder pad 74 connects the capacitor active via 122 at the first side 18A of the dielectric substrate 18 to the active conductive pathway 122 at the device side of the insulator 100, and a ground solder pad 86 connects the capacitor ground via 28 at the first side 18A of the dielectric substrate 18 to the ground conductive pathway 124 at the device side of the insulator 100. An insulative washer 68 resides between the device side of the insulator 50 and the first side 18A of the dielectric substrate 18 for the filter capacitor 14.

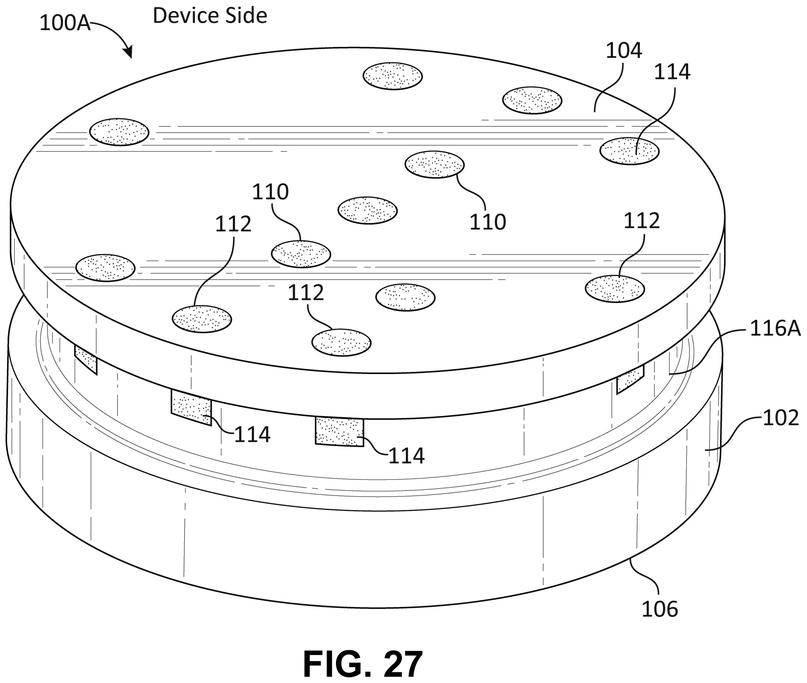

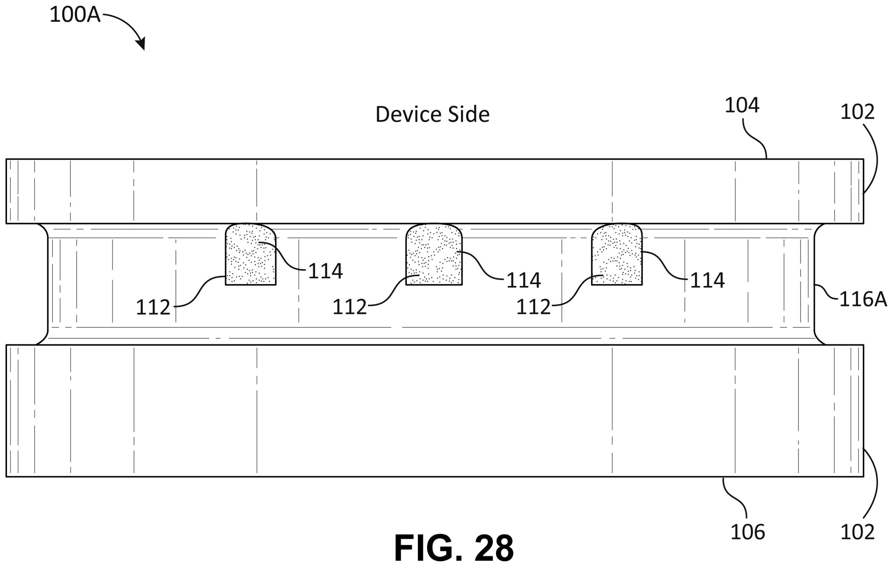

FIGS. 27 and 28 illustrate another exemplary insulator 100A showing an annular undercut 116A that extends completely around the perimeter of the insulator 100A. In other words, the annular undercut 116A is endless and effectively isolates the gold braze contacting the conductive pathways 114 in the ground holes 112 to prevent gold from reaching the solder pads 74, 76 (FIG. 20). Thus, according to the present invention the provision of an undercut in the sidewall of a ceramic insulator 100, 100A, whether a discrete undercut 116 or an annular undercut 116A, eliminates the possibility of gold braze material alloying with the material comprising the solder pads 74, 76 and the consequential detrimental effects on electrical conductivity.

It is appreciated that various modifications to the inventive concepts described herein may be apparent to those skilled in the art without departing from the spirit and scope of the present invention as defined by the hereinafter appended claims.

## Claims

1. An insulator, comprising:
a) a thickness defined by an insulator sidewall extending to an insulator device side opposite an insulator body fluid side;
b) at least one active conductive pathway extending through the insulator thickness to the insulator device and body fluid sides;
c) at least one ground conductive pathway extending part-way through the insulator thickness from the insulator device side; and
d) an undercut extending into the insulator sidewall so that the undercut is in communication with the ground conductive pathway.

2. The insulator of claim 1, wherein the undercut does not extend to the device or body fluid sides of the insulator.

3. The insulator of claim 1 or claim 2, wherein the undercut is not in communication with the active conductive pathway.

4. The insulator of any of claims 1 to 3, wherein the insulator has a plurality of ground conductive pathways, and wherein a plurality of discrete undercuts extending into the insulator sidewall are in communication with a respective one of the plurality of ground conductive pathways.

5. The insulator of any of claims 1 to 3, wherein the insulator has a plurality of ground conductive pathways, and wherein an annular undercut extends into the insulator sidewall in communication with each of the plurality of ground conductive pathways.

6. The insulator of any of claims 1 to 5, wherein a metallization is contacted to the insulator sidewall including the undercut, and wherein the metallization in the undercut contacts the at least one ground conductive pathway.

7. The insulator of claim 6, wherein the metallization extends to the device side of the insulator but does not extend to the body fluid side.

8. A feedthrough, comprising:
a) a ferrule defining a ferrule opening extending to a ferrule device side opposite a ferrule body fluid side;
b) an insulator as defined in any of claims 1 to 7 hermetically sealed to the ferrule in the ferrule opening, the insulator device side residing at or adjacent to the ferrule device side and the insulator body fluid side residing at or adjacent to the ferrule body fluid side; and
c) a braze hermetically sealing the insulator sidewall to the ferrule.

9. The feedthrough of claim 8, wherein the braze extends into the undercut so that the braze in in contact with the at least one ground conductive pathway.

10. An internally grounded filter feedthrough, comprising:
a) a feedthrough as defined in claim 8 or claim 9,
b) a filter capacitor, comprising:
i) a dielectric substrate supporting at least one active electrode plate interleaved in a capacitive relationship with at least one ground electrode plate, wherein the dielectric substrate has a substate sidewall extending to a first end surface opposite a second end surface;
ii) a capacitor active via extending through the dielectric substrate to the first and second end surfaces, wherein the active electrode plate is electrically connected to the capacitor active via; and
iii) a capacitor ground via extending through the dielectric substrate to the first and second end surfaces, wherein the ground electrode plate is electrically connected to the capacitor ground via,
c) wherein the dielectric substrate first end surface is positioned adjacent to the insulator device side with the capacitor active via connected to the active conductive pathway extending through the insulator so that an active electrical path extends from the capacitor active via at the second side of the dielectric substrate to the active conductive pathway at the body fluid side of the insulator, and
d) wherein the braze hermetically sealing the insulator sidewall to the ferrule also extends into the insulator undercut to contact the ground conductive pathway so that a ground electrical path extends from the capacitor ground via at the second side of the dielectric substrate to the ground conductive pathway in the insulator connected to the braze sealed to the ferrule.

11. The internally grounded filter feedthrough of claim 10, wherein an active solder pad connects the active via at the first side of the dielectric substrate to the active conductive pathway at the device side of the insulator, and wherein a ground solder pad connects the ground via at the first side of the dielectric substrate to the ground conductive pathway at the device side of the insulator.

12. The internally grounded filter feedthrough of claim 10 or claim 11, wherein an insulative washer resides between the device side of the insulator and the first side of the dielectric substrate for the filter capacitor.
